# EUROPEAN PATENT SPECIFICATION

(11) **EP 2 516 368 B1**
(45) Date of publication and mention of the grant of the patent: **01.07.2015**
(21) Application number: 10814773.7
(22) Date of filing: 22.12.2010
(51) Int. Cl.: C07C 21/18, C07C 17/087, C07C 17/20, C07C 17/25, C07C 17/42, C07C 19/10, B01J 37/26, B01J 23/86

(54) **CATALYTIC GAS PHASE FLUORINATION OF 1230XA TO 1234YF**
KATALYTISCHE GASPHASENFLUORIERUNG VON 1230XA ZU 1234YF
FLUORATION CATALYTIQUE EN PHASE GAZEUSE DU 1230XA DONNANT DU 1234YF

(30) Priority: 23.12.2009 WO PCT/IB2009/056054
(43) Date of publication of application: 31.10.2012
(73) Proprietor: Arkema France, 92700 Colombes (FR)
(72) Inventor: PIGAMO, Anne, F-69340 Francheville (FR); DEUR-BERT, Dominique, F-69390 Charly (FR); WENDLINGER, Laurent, F-69510 Soucieu En Jarrest (FR)
(74) Representative: Hirsch & Associés
(86) International application number: PCT/IB2010/056022
(87) International publication number: WO 2011/077394

(56) References cited:
- EP-A1- 0 939 071
- WO-A1-2009/003084
- WO-A1-2009/158321
- US-A1- 2009 030 245

## Description

### FIELD OF THE INVENTION

The present invention relates to the gas phase catalyzed fluorination of 1,1,2,3-tetrachloropropene (1230xa) to produce 2,3,3,3-tetrafluoropropene (1234yf). More particularly, the present invention relates to processes wherein 1230xa; optionally containing a low level of polymerization inhibitor, is contacted with hydrogen fluoride (HF) in a gas phase reaction, in the presence of a fluorination catalyst to produce 1234yf. The desired product, 1234yf is known to have utility as a foam blowing agent, refrigerant, aerosol propellant, heat transfer media, fire extinguisher, etc. Furthermore, 1234yf is known to have zero Ozone Depletion Potential (ODP) and very low Global Warming Potential (GWP) of much less than 150.

### BACKGROUND OF THE INVENTION

The Montreal Protocol for the protection of the ozone layer mandated the phase out of the use of chlorofluorocarbons (CFCs). Materials more "friendly" to the ozone layer, such as hydrofluorocarbons (HFCs) e.g. HFC-134a replaced chlorofluorocarbons. The latter compounds have proven to be green house gases, causing global warming and were regulated by the Kyoto Protocol on Climate Change. With the continued concern over global climate change there is an increasing need to develop technologies to replace those with high ozone depletion potential (ODP) and high global warming potential (GWP). Though hydrofluorocarbons (HFCs), being non-ozone depleting compounds, have been identified as alternatives to chlorofluorocarbons (CFCs) and hydrochloro-fluorocarbons (HCFCs) as solvents, cleaning agents and heat transfer fluids, they still tend to have significant GWP. Hydrofluoroolefins (HFO) have been identified as potential alternatives with zero ODP and low GWP.

Hence, numerous documents have provided such HFOs.

Methods of preparing hydrofluoroalkenes are known. For example, WO2007/079431 discloses processes for the production of fluorinated olefins, including hydrofluoropropenes. The processes which are broadly described as a single reaction or two or more reactions involve fluorination of compound of the formula C(X)ₘCCl(Y)ₙC(X)ₘ to at least one compound of formula CF₃CF=CHZ, where each X, Y and Z is independently H, F, Cl, I or Br and each m is independently 1, 2 or 3 and n is 0 or 1. 1234yf is prepared by fluorinating 1233xf into 1,1,1,2-tetrafluoro-2-chloropropane (HFC244bb), followed by dehydrochlorination. 1233xf is prepared by trifluorination of the corresponding chlorinated precursor (CCl₂=CClCH₂Cl).

EP-A-939071 discloses, among many possibilities, gas-phase fluorination of an halogenated propene (including in the list 1230xa) into a fluorinated propene (including in the list 1234yf).

WO2008/054781 discloses a variety of processes for producing a variety of fluoropropanes and halofluoropropenes by reacting halopropanes or halopropenes with HF optionally in the presence of a catalyst. It discloses a process for making 1234yf by reacting 2,3-dichloro-1,1,1-trifluoropropane (243db) in the presence of HF, on a catalyst, especially Cr/Co 98/2. Reaction products comprise 1234yf and 2-chloro-3,3,3-trifluoro-1-propene (1233xf), the latter being the main product; other products being 1-chloro-3,3,3-trifluoro-1-propene (1233zd) as well as 245cb and 1234ze which are also formed.

WO2008/002500 discloses a process for making a mixture of 2,3,3,3-tetrafluoro-1-propene (HFO 1234yf) and 1,3,3,3-tetrafluoro-1-propene (HFO 1234ze) by catalytic conversion of 1,1,1,2,3-pentafluoropropane (HFC 245eb) on a dehydrofluorination catalyst.

WO2008/040969 discloses a process comprising dehydrochlorination of 243db into 1233 (xf as well as zd), followed by a reaction involving formation of 1,1,1,2-tetrafluoro-2-chloropropane (244bb) and later formation of the desired 2,3,3,3-tetrafluoro-1-propene through dehydrochlorination. Example 1 of said document discloses a gas phase reaction at atmospheric pressure of 243db with HF on a Zn/chromia catalyst, whereby 1234yf and 1233xf are formed, together with a small amount of 245cb. Example 2 of said document discloses a gas phase reaction at atmospheric pressure of 245cb in presence of HF on the same catalyst (contact time 5 sec) whereby 1234yf is formed.

WO2009/015317 discloses the reaction of a chlorinated compound which can be 1,1,2,3-tetrachloro-1-propene (1230xa), 1,1,1,2,3-pentachloropropane (240db) or 2,3,3,3-tetrachloro-1-propene (1230xf) with HF, in gas phase, on a catalyst and in the presence of at least one stabilizer. This process allows obtaining 2-Chloro-3,3,3-trifluoro-1-propene (1233xf).

US2009/0240090 discloses a process for making 2,3,3,3-tetrafluoro-1-propene (1234yf) starting from a compound of formula (I) CX₂=CClCH₂X, or formula (II) CX₃CCl=CH₂ or formula (III) CX₃CHClCH₂X with X = F, Cl, Br, I. The process comprises three steps, which can be followed by purification. The process includes recycling steps allowing higher conversions and yields.

WO 2009/003084 discloses a process for the synthesis of hydrocchlorofluoro olefins (HCFO) and/or hydrofluoroolefins (HFO). The process of said document is based on the following steps of liquid phase, noncatalytic fluorination of hydrochloropropenes to form hydrochlorofluoropropenes and/or hydrofluoropropenes, followed by gas phase, noncatalytic fluorination of the hydrochlorofluoropropenes to form hydrofluoropropenes.

EP 0 939 071 discloses a method for producing a fluorinated propane. This method includes the steps of (a) fluorinating a halogenated propane and/or a halogenated propene by hydrogen fluoride in a gas phase in the presence of a first fluorination catalyst, thereby to obtain a reaction gas containing a fluorinated propene; and (b) fluorinating the fluorinated propene by hydrogen fluoride in a gas phase by transferring the reaction gas obtained by the step (a) to a reaction zone in which a second fluorination catalyst having an activated carbon supporting thereon a halide of a high valence metal is present, thereby to obtain the fluorinated propane.

US 2009/030245 discloses a method for preparing 2,3,3,3-tetrafluoropropene comprising contacting a reactant comprising CC12=CFCH2Cl with a fluorinating agent, such as HF, under conditions effective to produce a reaction product comprising CF3CF=CH2.

WO 2009/158321 is considered as comprised in the state of the art under Article 54(3) EPC. This document discloses a one reactor, gas phase catalyzed process for the fluorination of 1,1,2,3-tetrachloropropene (1230xa) to produce 1,1,1,2-tetrafluoropropene (1234yf). The process of this document is a catalytic, gas phase fluorination using a high pressure activated catalyst which is supported or unsupported, wherein fluorination products of the formula CF3R, where R is selected from -CCl=CH2, -CF=CH2, -CF2-CH3, -CFCl-CH3 and mixtures thereof are produced; co-produced materials are separated from the desired product and recycled to the same reactor.

### SUMMARY OF THE INVENTION

The invention is thus directed to a process for preparing 2,3,3,3-tetrafluoropropene (1234yf), comprising :
(i) contacting 1,1,2,3-tetrachloropropene (1230xa) with hydrogen fluoride HF in gas phase in the presence of a fluorination catalyst under conditions sufficient to produce a reaction mixture;
(ii) separating the reaction mixture into a first stream comprising HCl, 2,3,3,3-tetrafluoropropene (1234yf) and a second stream comprising HF, 2-chloro-3,3,3-trifluoro-1-propene (1233xf) and 1,1,1,2,2-pentafluoropropane (245cb);
(iii) recycling at least a part of the second stream at least in part back to step (i).

Embodiments are the following:
- the reaction mixture obtained at step (i) comprises 2,3,3,3-tetrafluoropropene (1234yf) and 1,1,1,2,2-pentafluoropropane (245cb) in a molar ratio of 1:5 to 3:1, preferably 1:4 to 2:1.
- the reaction mixture obtained at step (i) comprises 2,3,3,3-tetrafluoropropene (1234yf), 1,1,1,2,2-pentafluoropropane (245cb) and 1,1,1,2-tetrafluoro-2-chloropropane (244bb) such that the molar ratio of 245cb to 244bb is from 1:1 to 70:1, preferably from 1.5:1 to 65:1.
- step (i) is carried out with a molar ratio HF:1230xa from 3:1 to 150:1, preferably 4:1 to 70:1, more preferably 5:1 to 50:1.
- step (i) is carried out at a pressure from 3 to 20 bars, preferably 5 to 15 bars, more preferably 7 to 10 bars.
- step (i) is carried out at a temperature of from 200 to 450°C, preferably from 300 to 430°C, more preferably from 320 to 420°C.
- step (i) is carried out with a contact time from 6 to 100 sec, preferably from 10 to 80 sec, more preferably from 15 to 50 sec.
- step (i) is carried out in the presence of O₂ and/or Cl₂.
- the ratio of O₂ and/or Cl₂ with respect to 1,1,2,3-tetrachloropropene (1230xa) is 0.05 to 15 mole%, preferably 0.5 to 10 mole%.
- step (ii) is a distillation step.
- the first stream is further separated into HCl and 2,3,3,3-tetrafluoropropene (1234yf), preferably in a distillation step.
- step (i) is carried out in the presence of a polymerization inhibitor, preferably chosen from the group consisting of p-methoxyphenol, t-amylphenol, limonene, d,1-limonene, quinones, hydroquinones, epoxides, amines and mixtures thereof.
- step (i) is carried out in the presence of a catalyst comprising Ni-Cr, preferably supported.
- said catalyst is supported on a support selected from fluorinated alumina, fluorinated chromia, fluorinated activated carbon or graphite carbon.
- step (i) is carried out in the presence of a catalyst which is a chromium catalyst, supported or unsupported, preferably unsupported.
- said catalyst further comprises a co-catalyst selected from Ni, Co, Zn, Mn, Mg or mixtures thereof, preferably nickel and magnesium, and wherein said co-catalyst is preferably present in an amount from about 1-10 wt% of said fluorination catalyst.
- said fluorination catalyst is activated with a fluorine-containing compound, preferably hydrogen fluoride, and preferably at a pressure above 10 bars.
- the process is continuous.

The invention is also directed to a composition containing 2,3,3,3-tetrafluoropropene (1234yf), 1,1,1,2,2-pentafluoropropane (245cb) and 1,1,1,2-tetrafluoro-2-chloropropane (244bb) such that the molar ratio of 245cb to 244bb is from 1:1 to 70:1, and the molar ratio of 1234yf:245cb is from 1:5 to 3:1.

Embodiments are the following:
- the molar ratio of 245cb to 244bb is from 1.5:1 to 65:1, and the molar ratio of 1234yf:245cb is from 1:4 to 2:1.
- the composition further contains 2-chloro-3,3,3-trifluoro-1-propen (1233xf) and optionally HF and HCl.

### BRIEF DISCLOSURE OF THE DRAWINGS

Figure 1 is a scheme representing the possible reactions involved in the present invention.
Figure 2 is a scheme representing the process carried out in the invention.

### DETAILED DESCRIPTION OF EMBODIMENTS OF THE INVENTION

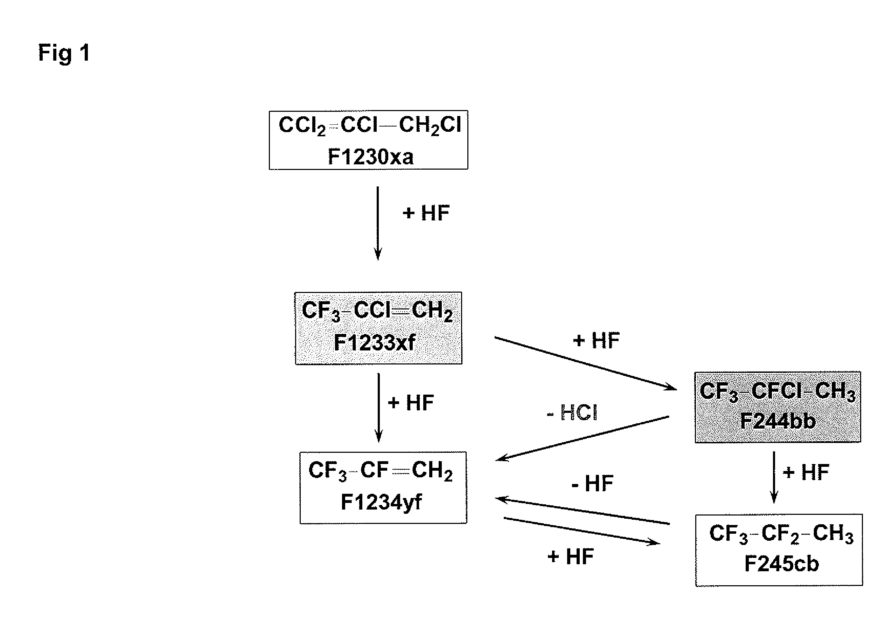

Figure 1 provides a scheme of potential reactions involved in the instant process. The first reaction is the hydrofluorination of 1230xa into 1233xf. Then 1233xf undergoes a series of possible reactions. Reaction with HF can lead directly to 1234yf or through an addition reaction to a saturated compound 244bb. This saturated compound can be dehydrochlorinated to give 1234yf. 244bb can also, upon fluorination with HF, give 245cb. 1234yf and 245cb form an equilibrium; the invention is based on this finding. Any 245cb formed can be recycled to the first reaction zone, whereby the equilibrium is displaced (1234yf being thus prevented from further conversion into 245cb). The 245cb flowrate in the recycling loop (either at the exit of the gas-phase reactor or in the second stream back exiting the distillation column and back to the gas-phase reactor) is thus substantially constant. No 245cb build up will thus take place in the recycling loop. In this instance, 1230xa fed into the reactor converts only into 1233xf and 1234yf since 245cb is already present in the recycling loop.

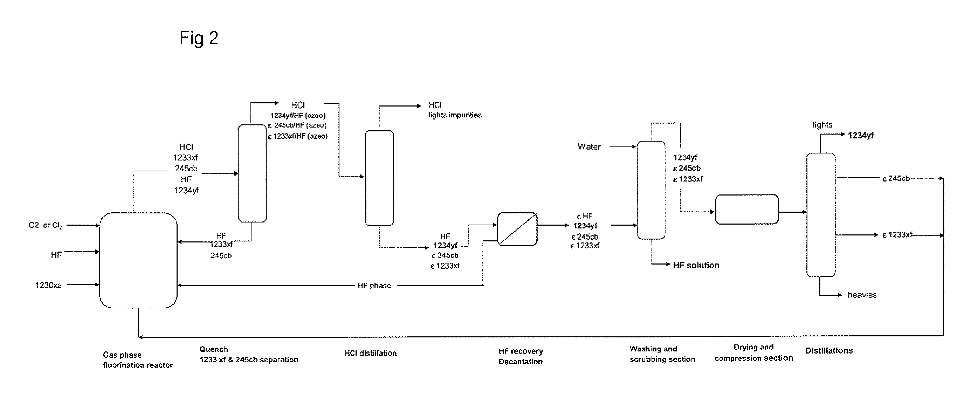

Figure 2 represents the process carried out in the invention. The gas-phase reactor is fed with 1230xa and HF. The reaction mixture exiting the reactor comprises HCl, 1233xf, unreacted HF, 1234yf, 245cb and a minor amount of 244bb. This reaction stream is separated by distillation into a first stream (light products) comprising HCl, 1234yf (possibly with a small amount of HF thereby forming an azeotropic mixture) and minor amounts of 245cb and 1233xf. A second, heavier, stream is obtained at the bottom of the distillation column, and comprises HF, 1233xf, 245cb and minor amounts of 244bb. The lighter fraction containing HCl, 1234yf (with HF) and minor amounts other products is again distillated. The top flow comprises HCl, while the bottom flow comprises 1234yf and HF, which can again be separated using appropriate known methods. Among known methods is the decantation, which produces an HF rich flow which can be recycled to the gas-phase reactor. This decreases the fluorine content downstream in the process, generating less side-products (e.g. CaF₂ which must be discarded). The streams exiting the decantation are treated according to known methods, including washing and scrubbing and distillations.

The applicant has found that the stream exiting the gas-phase reactor has a specific composition, due to the recycling of 245cb. Hence, the invention provides also a composition containing 2,3,3,3-tetrafluoropropene (1234yf), 1,1,1,2,2-pentafluoropropane (245cb) and 1,1,1,2-tetrafluoro-2-chloropropane (244bb) such that the molar ratio of 245cb to 244bb is from 1:1 to 70:1, and the molar ratio of 1234yf:245cb is from 1:5 to 3:1; preferably the molar ratio of 245cb to 244bb is from 1.5:1 to 65:1, and the molar ratio of 1234yf:245cb is from 1:4 to 2:1. The composition may also comprise 1233xf, and optionally other compounds such as unreacted HF and HCl.

The gas phase reaction is carried out in the presence of a fluorination catalyst. The reaction is carried out in a single gas-phase reactor.

The level of the conversion and selectivity of the desired product can vary according to the processing conditions. The catalyst can be present in any suitable form, such as fixed or fluidized bed, preferably in a fixed bed. The direction of flow may be downward or upward.

This catalyst is for example a catalyst based on a metal including a transition metal oxide or a derivative or halide or oxyhalide such a metal. Catalysts are e.g. FeCl₃, chromium oxyfluoride, chromium oxides (that can optionally be subject to fluorination treatments), chromium fluorides, and mixtures thereof. Other possible catalysts are the catalysts supported on carbon catalysts based on antimony, catalysts based on aluminum (as AlF₃ and Al₂O₃ and oxyfluoride of alumina and aluminum fluoride). Generally speaking, catalysts that can be used are chromium oxyfluoride, aluminium fluorure and oxyfluoride, and supported or unsupported catalyst containing a metal such as Cr, Ni, Zn, Ti, V, Zr, Mo, Ge, Sn, Pb, Mg. Reference can also be made to the disclosures of WO-A-2007/079431, at page 7, lines 1-5 and 28-32, EP-A-939071, at paragraph [0022], WO2008/054781 at page 9 line 22 to page 10 line 34, WO2008/040969 in claim 1, all incorporated herein by reference.

Prior to its use, the catalyst is subjected to activation with HF at high pressure, typically above about 10 bars (typically at a pressure above the pressure used in the gas-phase process), as described in US-A-7485598, incorporated herein by reference.

A preferred embodiment uses a particular catalyst, which is a mixed catalyst, containing both chromium and nickel. The molar ratio Cr: Ni, with respect to the metallic element is generally between 0.5 and 5, for example between 0.7 and 2, including close to 1. The catalyst may contain in weight from 0.5 to 20% chromium and 0.5 to 20% nickel, preferably between 2 and 10% of each metal.

The metal may be present in metallic form or as derivatives, including oxide, halide or oxyhalide. These derivatives, including halide and halide oxides, are obtained by activation of the catalytic metal. Although the activation of the metal is not necessary, it is preferred.

The support is preferably made from aluminum. There are several possible carriers such as alumina, activated alumina or aluminum derivatives. These derivatives include aluminum halides and halide oxides of aluminum, for example described in US-P-4902838, or obtained by the activation process described below.

The catalyst may include chromium and nickel in a non-activated or activated form, on a support that has been subjected to activation or not.

Reference can be made to WO2009/118628, and especially to the disclosure of the catalyst from page 4, line 30 to page 7, line 16, which is incorporated herein by reference.

According to another embodiment, the process uses a high surface area Cr based catalyst which is preferably unsupported. The catalyst can optionally contain a low level of one or more co-catalyst such as Co, Zn, Mn, Mg and Ni salt. A preferred co-catalyst is nickel. Another preferred co-catalyst is Zn.

The preferred unsupported chromium catalyst can optionally contain low levels of one or more co-catalysts selected from cobalt, nickel, zinc, manganese or magnesium, prepared by processes known in the art, such as impregnation, mixed powder and the like. The catalyst can be supported or unsupported. For supported catalyst, the catalyst support can be selected from materials known in the art to be compatible with HF at higher temperature and pressure. For example, fluorinated alumina, prefluorinated activated carbon, graphite or fluorinated graphite are suitable catalyst supports. A preferred chromium catalyst is a high surface area unsupported chromium oxide catalyst. The catalyst is activated before use. The catalyst activation typically comprises a high pressure, above 10 bars, procedure wherein the catalyst bed is heated to about 370-380° C, preferably with a continuous flow of nitrogen, after which a mixture of approximately equal volumes of HF and air or nitrogen (preferably nitrogen) are fed over the catalyst bed. The catalyst activation process can be as described in US7485598, incorporated herein by reference. Other fluorinated organic compounds such as CHF₂Cl, CHF₃, CF₃CH₂F, CF3CH2Cl and the like can be used for activation. Typically the high pressure catalyst activation procedure takes about 18 hours.

The resulted high-pressure activated catalyst has a high surface area, such as from about 20 to about 250 square meters per gram. The fluorine content typically varies between about 20 to 25 wt%. The pore volume has an average value between 0.1 to 0.4 m³/g. Crushing strength is typically between about 8 to 15 kg/g. Percent attrition is typically on average between 1 to 5 wt%. Cr ^{(VI)} level is typically in the range of 100 to 300 ppm.

The level of the co-catalyst, when present, can be varied between 1 to 10 wt%, preferable between 1 to 5 wt%. Co-catalyst can be added to the catalyst by processes known in the art such as adsorption from an aqueous or organic solution, followed by solvent evaporation. The preferred catalyst in this embodiment is pure chromium oxide with nickel or zinc as a co-catalyst. Alternatively the co-catalyst can be physically mixed with the catalyst via grinding to produce an intimate mixture. An alternative catalyst is a mixed chromium/nickel catalyst supported on fluorinated alumina. US5731481, incorporated herein by reference, discloses a method of preparation of this alternative catalyst which would be activated as described hereinabove.

For example, a catalyst which can be used in the invention can be prepared as follows. A Cr₂O₃ catalyst was activated at 16 bars and 350° C using HF and nitrogen gas. The chemical and physical properties of the resulting catalyst are shown in the table below.

| | |
|---|---|
| %F Content wt% | 22.2 |
| Surface Area m²/g¹ | 43.9 |
| Pore Volume m³/g² | 0.19 |
| Crush Strength kg/g³ | 10.6 |
| Cr^{+VI} content ppm | 201 |
| % Attrition⁴ | 3.9 |

| | |
|---|---|
| 1. Surface area was determined by the BET surface area by Micrometrics ASAP 2400 2. Pore volume was evaluated using xylene porosity measurement. 3. Crush strength was evaluated by applying a specified rate of compression, until the integrity of the catalyst is compromised. 4. Percent Attrition was evaluated by using ASTM D-4058-92 Standard test method for attrition. | |

The process of the present invention is preferably run continuously.

The 1230xa fluorination process involves contacting 1230xa with HF in the reaction zone in a gas phase, under conditions sufficient to convert the 1230xa to fluorination products comprising 1233xf, 1234yf and 245cb. Such conditions are given below. In addition, other co-produced underfluorinated intermediates such as 1233xf (and 244bb which may be present in minor amounts) which are produced as part of the fluorination reaction are also recycled to the reactor. The recycle stream contains the heavy fraction of the reaction stream which has been separated in the distillation step, and especially the equilibrated 245cb.

Typically, step (i) is carried out with a molar ratio HF:1230xa from 3:1 to 150:1, preferably 4:1 to 70:1, more preferably 5:1 to 50:1.

Typically, step (i) is carried out at a pressure from 3 to 20 bars, preferably 5 to 15 bars, more preferably 7 to 10 bars.

Typically, step (i) is carried out at a temperature of from 200 to 450°C, preferably from 300 to 430°C, more preferably from 320 to 420°C. The temperature of the bed can be substantially uniform in the reactor or can be adjusted along the path of the stream, decreasing or increasing along the direction of flow.

Contact times (catalyst volume divided by the total flow rate of reactants and co-feeds, adjusted to the operating pressure and temperature) are typically from 6 to 100 sec, preferably from 10 to 80 sec, more preferably from 15 to 50 sec.

An oxygen co-feed or chlorine co-feed can be used to extend the catalyst lifetime, typically in an amount of from 0.05 to 15 mole%, preferably 0.5 to 10 mole% of oxygen or chlorine per 1230xa. The oxygen can be introduced as an oxygen-containing gas such as air, pure oxygen, or an oxygen/nitrogen mixture. Chlorine can be introduced as a chlorine-containing gas such as pure chlorine, or a chlorine/nitrogen mixture.

A polymerization inhibitor can be used to extend the catalyst life, typically in a concentration of from about 50-1000 ppm, more preferably between 100-500 ppm. The polymerization inhibitor can be p-methoxyphenol, t-amylphenol, limonene, d,1-limonene, quinones, hydroquinones, epoxides, amines and mixtures thereof. The preferred polymerization inhibitor is p-methoxyphenol or t-amylphenol. The co-feeding of a low level of a polymerization inhibitor can control such polymerization of chloroolefins and extend the life of the catalyst as described in US5714651, incorporated herein by reference.

Conversion of 1230xa is typically higher than 90%, preferably higher than 95% and more preferably higher than 97%.

The reactants can be fed to the reactor at the same location, at different locations, or using staged feeding at staged locations along the reactor. A preferred feeding system is to blow the gaseous reactants at the bottom of the reactor. Recycling can be done at the entry of the reactor or at an intermediate stage of the reactor; preferably at the entry of the reactor.

In another embodiment, the reaction stream exiting the gas-phase reactor can be recycled in part to the reactor, before it is subjected to the separation into a first, light, stream and a second, heavy stream. The recycling ratio can be as high as 0.7. This recycling allows dilution of 1230xa which is very reactive and avoids polymerisation.

Reactions are implemented in a dedicated reactor for reactions involving halogens. Such reactors are known to those skilled in the art and can include linings based eg Hastelloy®, Inconel®, Monel® or fluoropolymers. The reactor may also include means of heat exchange, if necessary.

As used herein, percentages are by molar percent unless specified otherwise.

The following examples illustrate the invention without limiting it.

### EXAMPLES.

In the following examples, use is made of a catalyst Ni-Cr/AlF₃ which is obtained as follows. The catalyst used is a mixed catalyst nickel/chromium of atomic ratio of Ni/Cr = 1, supported on alumina fluoride and is prepared by impregnating solutions of nickel and chromic anhydride (CrO₃). After impregnation and drying, the solid is treated at a temperature between 320°C and 390°C in the presence of a mixture of hydrofluoric acid and nitrogen (concentration by volume of 5 to 10% of this acid in nitrogen). The catalyst bed is placed on a grid welded to the lower end of reactor. The reactor is equipped with a temperature measurement at three locations along the catalyst bed.

### Examples 1 to 5. Equilibrium 1234yf/245cb.

150 ml of catalyst Ni-Cr/AlF₃ are introduced into the reactor. The reaction temperature is 352°C and atmospheric pressure. The flows of HF and 1233xf are adjusted to obtain a molar ratio HF/1233xf close to 5 but varying contact time: 9.5, 18.8, 26.7, 37.9 and 39 seconds. The products that are mainly obtained are 1234yf and 245cb (if one excludes the 1233xf, not completely converted). The molar compositions 1234yf and 245cb are summarized in table 1. It can be concluded that an equilibrium takes place between 1234yf and 245cb.

| | Contact time | 1234yf | 245cb |
|---|---|---|---|
| | (s) | (%) | (%) |
| Ex. 2 | 9.5 | 72.53 | 27.47 |
| Ex. 3 | 18.8 | 69.13 | 30.87 |
| Ex. 4 | 26.7 | 66.49 | 33.51 |
| Ex. 5 | 37.9 | 66.28 | 33.72 |
| Ex. 6 | 39 | 66.18 | 33.82 |

### Example 6. Fluorination of 1230xa.

It uses a reactor consisting of an inconel® tube of internal diameter 28 mm and length of 640 mm, placed vertically in a tubular furnace. The catalyst bed consists of a lower layer of 40 mm corundum, and a catalyst layer of 85 mm (volume = 50cm3, mass = 42.1g). The reagents are introduced continuously at the bottom of the reactor and preheated the oven temperature. The gaseous products exit at the upper end of the reactor through a control valve; they are analyzed by gas chromatography.

The operating conditions in the reactor of fluorination are:
- Temperature 340°C;
- Absolute pressure 7 bar;
- Molar ratio HF/1230xa = 40;
- Molar ratio O₂/(HF + 1230xa) = 0.1%
- Contact time = 11 sec and 22 sec.

After 200 hours of reaction, the result of analysis of gaseous products (outside hydracids) from the reactor is given in the following table:

| | Contact time 11 sec | Contact time 22 sec |
|---|---|---|
| Conversion rate of 1230xa | 99.7 % | 100.0 % |
| Selectivity 1234yf | 0.45 % | 2.42 % |
| Selectivity 245cb | 0.29 % | 2.85 % |
| Selectivity 1233xf | 97.7 % | 92.7 % |
| Selectivity 244bb | 1.1 % | 1.1 % |
| Selectivity 243db | 0.1 % | 0.15 % |
| Selectivity « others » | 0.36 % | 0.78 % |

One will note that the content in 244bb is substantially constant; this compound appears to be an intermediate compound formed during the reaction between 1233xf and 1234yf (further to the reaction 1230xa into 1233xf) on the one hand and during the reaction with 1234yf and 245cb on the other hand. A higher contact time will favour formation of 1234yf, where the equilibrium with 245cb is reached.

## Claims

1. A process for preparing 2,3,3,3-tetrafluoropropene (1234yf), comprising :
(i) contacting 1,1,2,3-tetrachloropropene (1230xa) with hydrogen fluoride HF in gas phase in the presence of a fluorination catalyst under conditions sufficient to produce a reaction mixture;
(ii) separating the reaction mixture into a first stream comprising HCl, 2,3,3,3-tetrafluoropropene (1234yf) and a second stream comprising HF, 2-chloro-3,3,3-trifluoro-1-propene (1233xf) and 1,1,1,2,2-pentafluoropropane (245cb);
(iii) recycling at least a part of the second stream at least in part back to step (i).

2. Process according to claim 1, wherein the reaction mixture obtained at step (i) comprises 2,3,3,3-tetrafluoropropene (1234yf) and 1,1,1,2,2-pentafluoropropane (245cb) in a molar ratio of 1:5 to 3:1.

3. Process according to claim 1 or 2, wherein step (i) is carried out with a molar ratio HF:1230xa from 3:1 to 150:1.

4. Process according to any one of claims 1 to 3, wherein step (i) is carried out at a pressure from 3 to 20 bars, and/or at a temperature of from 200 to 450°C.

5. Process according to any one of claims 1 to 4, wherein step (i) is carried out with a contact time from 6 to 100 sec.

6. Process according to any one of claims 1 to 5, wherein step (i) is carried out in the presence of O₂ and/or Cl₂.

7. Process according to any one of claims 1 to 6, wherein step (ii) is a distillation step.

8. Process according to any one of claims 1 to 7, wherein the first stream is further separated into HCl and 2,3,3,3-tetrafluoropropene (1234yf).

9. Process according to any one of claims 1 to 8, wherein step (i) is carried out in the presence of a polymerization inhibitor.

10. Process according to any one of claims 1 to 9, wherein step (i) is carried out in the presence of a catalyst comprising Ni-Cr.

11. Process according to any one of claims 1 to 9, wherein step (i) is carried out in the presence of a catalyst which is a chromium catalyst, supported or unsupported.

12. Process according to any one of claims 1 to 11, wherein said fluorination catalyst is activated with a fluorine-containing compound.

13. The process according to any one of the preceding claims which is continuous.

## Patentansprüche

1. Verfahren zur Herstellung von 2,3,3,3-Tetrafluorpropen (1234yf), umfassend:
(i) Inkontaktbringen von 1,1,2,3-Tetrachlorpropen (1230xa) mit Fluorwasserstoff HF in Gasphase in der Gegenwart eines Fluorierungskatalysators unter Bedingungen, die geeignet sind, um ein Reaktionsgemisch herzustellen;
(ii) Trennen des Reaktionsgemischs in einen ersten Strom, umfassend HCl, 2,3,3,3-Tetrafluorpropen (1234yf) und einen zweiten Strom, umfassend HF, 2-Chlor-3,3,3-trifluor-1-propen (1233xf) und 1,1,1,2,2-Pentafluorpropan (245cb);
(iii) mindestens teilweises Zurückführen von mindestens einem Teil des zweiten Stroms zu Schritt (i).

2. Verfahren nach Anspruch 1, wobei das in Schritt (i) erhaltene Reaktionsgemisch (i) 2,3,3,3-Tetrafluorpropen (1234yf) und 1,1,1,2,2-Pentafluorpropan (245cb) in einem Molverhältnis von 1:5 bis 3:1 umfasst.

3. Verfahren nach Anspruch 1 oder 2, wobei Schritt (i) mit einem Molverhältnis HF:1230xa von 3:1 bis 150:1 durchgeführt wird.

4. Verfahren nach einem der Ansprüche 1 bis 3, wobei Schritt (i) bei einem Druck von 3 bis 20 bar und/oder bei einer Temperatur von 200 bis 450 °C durchgeführt wird.

5. Verfahren nach einem der Ansprüche 1 bis 4, wobei Schritt (i) mit einer Kontaktzeit von 6 bis 100 Sek. durchgeführt wird.

6. Verfahren nach einem der Ansprüche 1 bis 5, wobei Schritt (i) in Gegenwart von O₂ und/oder Cl₂ durchgeführt wird.

7. Verfahren nach einem der Ansprüche 1 bis 6, wobei Schritt (ii) ein Destillationsschritt ist.

8. Verfahren nach einem der Ansprüche 1 bis 7, wobei der erste Strom ferner in HCl und 2,3,3,3-Tetrafluorpropen (1234yf) getrennt wird.

9. Verfahren nach einem der Ansprüche 1 bis 8, wobei Schritt (i) in Gegenwart eines Polymerisationshemmers durchgeführt wird.

10. Verfahren nach einem der Ansprüche 1 bis 9, wobei Schritt (i) in Gegenwart eines Katalysators, umfassend Ni-Cr, durchgeführt wird.

11. Verfahren nach einem der Ansprüche 1 bis 9, wobei Schritt (i) in Gegenwart eines Katalysators durchgeführt wird, bei dem es sich um einen geträgerten oder ungeträgerten Chromkatalysator handelt.

12. Verfahren nach einem der Ansprüche 1 bis 11, wobei der Fluorierungskatalysator mit einer Fluor-enthaltenden Verbindung aktiviert wird.

13. Verfahren nach einem der vorhergehenden Ansprüche, das kontinuierlich ist.

## Revendications

1. Procédé de préparation de 2,3,3,3-tétrafluoropropène (1234yf), comprenant:
(i) mettre en contact du 1,1,2,3-tétrachloropropène (1230xa) avec du fluorure d'hydrogène HF en phase gazeuse en présence d'un catalyseur de fluoration dans des conditions suffisantes pour produire un mélange réactionnel ;
(ii) séparer le mélange réactionnel en un premier flux comprenant du HCl, du 2,3,3,3-tétrafluoropropène (1234yf) et un deuxième flux comprenant du HF, du 2-chloro-3,3,3-trifluoro-1-propène (1233xf) et du 1,1,1,2,2-pentafluoropropane (245cb);
(iii) recycler au moins une partie du second flux au moins en partie retournée à l'étape (i).

2. Procédé selon la revendication 1, dans lequel le mélange réactionnel obtenu à l'étape (i) comprend du 2,3,3,3-tétrafluoropropène (1234yf) et du 1,1,1,2,2-pentafluoropropane (245cb) dans un rapport molaire de 1:5 à 3:1.

3. Procédé selon la revendication 1 ou 2, dans lequel l'étape (i) est effectuée avec un rapport molaire HF:1230xa de 3:1 à 150:1.

4. Procédé selon l'une quelconque des revendications 1 à 3, dans lequel l'étape (i) est effectuée à une pression de 3 à 20 bars et/ou à une température de 200 à 450°C.

5. Procédé selon l'une quelconque des revendications 1 à 4, dans lequel l'étape (i) est effectuée avec un temps de contact de 6 à 100 secondes.

6. Procédé selon l'une quelconque des revendications 1 à 5, dans lequel l'étape (i) est effectuée en présence d'O2 et/ou de Cl₂.

7. Procédé selon l'une quelconque des revendications 1 à 6, dans lequel l'étape (ii) est une étape de distillation.

8. Procédé selon l'une quelconque des revendications 1 à 7, dans lequel le premier flux est en outre séparé en HCl et en 2,3,3,3-tétrafluoropropène (1234yf).

9. Procédé selon l'une quelconque des revendications 1 à 8, dans lequel l'étape (i) est effectuée en présence d'un inhibiteur de polymérisation.

10. Procédé selon l'une quelconque des revendications 1 à 9, dans lequel l'étape (i) est effectuée en présence d'un catalyseur comprenant Ni-Cr.

11. Procédé selon l'une quelconque des revendications 1 à 9, dans lequel l'étape (i) est effectuée en présence d'un catalyseur qui est un catalyseur au chrome, supporté ou non supporté.

12. Procédé selon l'une quelconque des revendications 1 à 11, dans lequel ledit catalyseur de fluoration est activé avec un composé contenant du fluor.

13. Procédé selon l'une quelconque des revendications précédentes qui est un procédé continu.
